# EUROPEAN PATENT APPLICATION

(11) **EP 4 174 868 A1**
(43) Date of publication of application: **03.05.2023**
(21) Application number: 21205754.1
(22) Date of filing: 01.11.2021
(51) Int. Cl.: G16H 30/40, G16H 50/70, A61B 5/055, G06N 3/02

(54) **SYNTHETIC CONTRAST-ENHANCED MR IMAGES**

(71) Applicant: Bayer Aktiengesellschaft, 51373 Leverkusen (DE); Universitätsklinikum Essen, 45147 Essen (DE)
(72) Inventor: The designation of the inventor has not yet been filed
(74) Representative: BIP Patents

(57) **Abstract**

Systems, methods, and computer programs disclosed herein relate to training and using a machine learning model to generate contrast-enhanced magnetic resonance images.

## Description

### FIELD

Systems, methods, and computer programs disclosed herein relate to training and using a machine learning model to generate contrast-enhanced magnetic resonance images.

### BACKGROUND

Medical imaging is the technique and process of imaging the interior of a body for clinical analysis and medical intervention, as well as visual representation of the function of some organs or tissues (physiology). Medical imaging seeks to reveal internal structures hidden by the skin and bones, as well as to diagnose and treat diseases.

Many types of medical imaging use contrast agents to enhance the visualization of normal and abnormal structures. Examples include conventional angiography, fluoroscopy, computed tomography (CT), ultrasound, and magnetic resonance imaging (MRI). It is sometimes desirable to reduce the contrast agent dose, however, reduced dose usually reduces desired imaging enhancements.

Advances in both imaging and machine learning have synergistically led to a rapid rise in the potential use of artificial intelligence in various medical imaging tasks, such as risk assessment, detection, diagnosis, prognosis, and therapy response.

Nowadays, machine learning is used not only for classification of images or detection of symptoms, but also for the generation of synthetic images. In particular, WO2019/074938A1 discloses that machine learning is able to predict a synthesized full-dose contrast agent image from a low-dose contrast agent image and a pre-dose image.

WO2019/074938A1 relates to a method and a system for performing diagnostic imaging of a subject with reduced contrast agent dose. In a first step, a set of diagnostic images of a set of subjects is produced. The set of images comprises, for each sample of the data set, i) a full-contrast image acquired with a full-contrast agent dose administered to the subject, ii) a low-contrast image acquired with a low-contrast agent dose administered to the subject, where the low-contrast agent dose is less than the full-contrast agent dose, and iii) a zero-contrast image acquired with no contrast agent dose administered to the subject. In a second step, a deep learning network (DLN) is trained by applying zero-contrast images from the set of images and low-contrast images from the set of images as input to the DLN and using a cost function to compare the output of the DLN with full-contrast images from the set of images to train parameters of the DLN using backpropagation. Once, the DLN is trained, it can be used to generate a synthesized full-contrast contrast agent image of a subject by applying a low-contrast image and a zero-contrast image as input to the trained DLN.

When comparing a synthesized full-contrast image generated in accordance with the method described in WO2019/074938A1 with the respective real full-contrast image, deviations can be observed; for example, some blurring artifacts may occur.

In light of the cited prior art, there is a need to provide new methods which improve the quality of synthesized full-contrast images. In particular, there is a need for methods which improve the quality of contrast-enhanced images which have not been acquired at full-dose to resemble full-contrast images. By improving the quality of contrast-enhanced images not taken at full dose to resemble full-contrast images, it is possible to reduce the amount of contrast agent employed, which are oftentimes expensive, cumbersome to handle and associated with undesired side effects. It is therefore possible to reduce the cost of the technique and to improve the experience for the patient undergoing the procedure.

Thus, the technical problem to be solved is to provide methods which further improve the quality of contrast-enhanced images. In this context, quality may be characterized by the ability of machine learning models to learn small details that may have little impact on global error metrics, but bring significant clinical value, such as reduced blurring artifacts.

### SUMMARY

This problem is solved by the subject matter of the independent claims of the present disclosure. Preferred embodiments of the present disclosure are defined in the dependent claims and described in the present specification and/or depicted in the figures.

In a first aspect, the present disclosure provides a method of training a machine learning model to generate a synthetic contrast-enhanced MR image that represents at least a portion of an examination region within an examination object, the method comprising the steps of:
- receiving a training data set, the training data set comprising, for each object of a multitude of objects, MR data, and target MR data,
   - wherein the MR data comprise a plurality of representations of a first examination region within the object after administration of a first dose of a contrast agent,
   - wherein the target MR data represent at least a portion of the first examination region within the object after administration of a second dose of a contrast agent, wherein the second dose is different from the first dose,
- training the machine learning model, thereby obtaining a trained machine learning mode, wherein the training comprises:
   ∘ inputting the MR data into the machine learning model,
      wherein the machine learning model is configured to generate, at least partially on the basis of the MR data and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
- outputting the trained machine learning model, and/or storing the machine learning model on a data storage, and/or providing the trained machine learning model for predictive purposes.

In another aspect, the present disclosure provides a computer system comprising:
a processor; and
a memory storing an application program configured to perform, when executed by the processor, an operation, the operation comprising:
   - receiving a training data set, the training data set comprising, for each object of a multitude of objects, MR data, and target MR data,
      - wherein the MR data comprise a plurality of representations of a first examination region within the object after administration of a first dose of a contrast agent,
      - wherein the target MR data represent at least a portion of the first examination region within the object after administration of a second dose of a contrast agent, wherein the second dose is different from the first dose,
   - training the machine learning model, thereby obtaining a trained machine learning mode, wherein the training comprises:
      ∘ inputting the MR data into the machine learning model,
         wherein the machine learning model is configured to generate, at least partially on the basis of the MR data and model parameters, predicted MR data,
      ∘ receiving from the machine learning model the predicted MR data,
      ∘ computing a loss value, the loss value quantifying the deviations between the predicted MR data and the target MR data,
      ∘ modifying one or more of the model parameters to minimize the loss value,
   - outputting the trained machine learning model, and/or storing the machine learning model on a data storage, and/or providing the trained machine learning model for predictive purposes.

In another aspect, the present disclosure provides a non-transitory computer readable medium having stored thereon software instructions that, when executed by a processor of a computer system, cause the computer system to execute the following steps:
- receiving a training data set, the training data set comprising, for each object of a multitude of objects, MR data, and target MR data,
   - wherein the MR data comprise a plurality of representations of a first examination region within the object after administration of a first dose of a contrast agent,
   - wherein the target MR data represent at least a portion of the first examination region within the object after administration of a second dose of a contrast agent, wherein the second dose is different from the first dose,
- training the machine learning model, thereby obtaining a trained machine learning mode, wherein the training comprises:
   ∘ inputting the MR data into the machine learning model,
      wherein the machine learning model is configured to generate, at least partially on the basis of the MR data and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
- outputting the trained machine learning model, and/or storing the machine learning model on a data storage, and/or providing the trained machine learning model for predictive purposes.

In another aspect, the present disclosure provides a kit, the kit comprising a contrast agent and a non-transitory computer-readable storage medium, the computer-readable storage medium having stored thereon software instructions that, when executed by a processor of a computer system, cause the computer system to execute the following steps:
- receiving a training data set, the training data set comprising, for each object of a multitude of objects, MR data, and target MR data,
   - wherein the MR data comprise a plurality of representations of a first examination region within the object after administration of a first dose of a contrast agent,
   - wherein the target MR data represent at least a portion of the first examination region within the object after administration of a second dose of a contrast agent, wherein the second dose is different from the first dose,
- training the machine learning model, thereby obtaining a trained machine learning mode, wherein the training comprises:
   ∘ inputting the MR data into the machine learning model,
      wherein the machine learning model is configured to generate, at least partially on the basis of the MR data and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
- outputting the trained machine learning model, and/or storing the machine learning model on a data storage, and/or providing the trained machine learning model for predictive purposes.

Further aspects of the present disclosure are described hereinafter.

### DETAILED DESCRIPTION

The invention will be more particularly elucidated below, without distinguishing between the aspects of the disclosure (methods, computer systems, computer-readable storage media, kits). On the contrary, the following elucidations are intended to apply analogously to all the aspects, irrespective of in which context (methods, computer systems, computer-readable storage media, kits) they occur.

If steps are stated in an order in the present description or in the claims, this does not necessarily mean that the disclosure is restricted to the stated order. On the contrary, it is conceivable that the steps can also be executed in a different order or else in parallel to one another, unless one step builds upon another step, this absolutely requiring that the building step be executed subsequently (this being, however, clear in the individual case). The stated orders are thus preferred embodiments of the invention.

As used herein, the articles "a" and "an" are intended to include one or more items and may be used interchangeably with "one or more" and "at least one." As used in the specification and the claims, the singular form of "a", "an", and "the" include plural referents, unless the context clearly dictates otherwise. Where only one item is intended, the term "one" or similar language is used. Also, as used herein, the terms "has", "have", "having", or the like are intended to be open-ended terms. Further, the phrase "based on" is intended to mean "based at least partially on" unless explicitly stated otherwise. Further, the phrase "based on" may mean "in response to" and be indicative of a condition for automatically triggering a specified operation of an electronic device (e.g., a controller, a processor, a computing device, etc.) as appropriately referred to herein.

Some implementations of the present disclosure will be described more fully hereinafter with reference to the accompanying drawings, in which some, but not all implementations of the disclosure are shown. Indeed, various implementations of the disclosure may be embodied in many different forms and should not be construed as limited to the implementations set forth herein; rather, these example implementations are provided so that this disclosure will be thorough and complete, and will fully convey the scope of the disclosure to those skilled in the art.

The present disclosure provides solutions for generating synthetic contrast-enhanced magnetic resonance (MR) images.

Magnetic resonance imaging, MRI for short, is an imaging method which is used especially in medical diagnostics for depicting structure and function of the tissue and organs in the human or animal body.

In MRI, the magnetic moments of protons in an examination object are aligned in a basic magnetic field, with the result that there is a macroscopic magnetization along a longitudinal direction. The magnetic moments of protons in the examination object are then deflected from a resting position (e.g., a relaxation position) by irradiation with high-frequency (HF) pulses (e.g., by excitation with HF pulses of electromagnetic radiation). The return of the protons from the excited states to the resting positions, or magnetization dynamics, may then be detected as relaxation signals by one or more HF receiver coils of an MRI machine. For spatial encoding, rapidly switched magnetic gradient fields may be superimposed on the basic magnetic field. The relaxation signals are initially present as raw data in frequency space (also referred to as k-space data) and can be transformed (e.g. by an inverse Fourier transform) into real space (e.g., image space). In one example, during the use of native MRI, tissue contrasts may be created by different relaxation times (e.g., the spin-lattice relaxation time and the spin-spin relaxation time, referred to as T1 and T2) and/or proton density. The spin-lattice relaxation time may describe the transition of the longitudinal magnetization to its equilibrium magnetization state, where spin-lattice relaxation time is measured as the time taken to reach 63.21% of the equilibrium magnetization prior to the resonance excitation. The spin-spin relaxation time may describe, in an analogous manner, the transition of transverse magnetization to its equilibrium magnetization state.

In MRI, contrast agents are commonly used. MRI contrast agents develop their action by altering the relaxation times of the structures which take up contrast agents. A distinction can be made between two groups of substances: paramagnetic and superparamagnetic substances. Both groups of substances have unpaired electrons which induce a magnetic field around the individual atoms or molecules.

Superparamagnetic contrast agents lead to a predominant shortening of T2, whereas paramagnetic contrast agents mainly lead to a shortening of T1. A shortening of the T1 time leads to an increase in the signal intensity in T 1-weighted MRI images, and a shortening of the T2 time leads to a decrease in the signal intensity in T2-weighted MRI images.

The action of said contrast agents is indirect, since the contrast agent itself does not give off a signal, but instead only influences the signal intensity of the hydrogen protons in its surroundings.

The present disclosure provides solutions for generating a synthetic contrast-enhanced MR image which represents an examination region of an examination object.

The "examination object" is usually a living being, preferably a mammal, very particularly preferably a human.

The "examination region" is a portion of the examination object, for example an organ or a portion of an organ. Preferably, the examination region is the liver or a portion of the liver of a mammal or the brain or a portion of the brain of a mammal (preferably a human). The examination region, also called image volume (field of view, FOV), is in particular a volume which is imaged in the magnetic resonance images. The examination region is typically defined by a radiologist, for example on an overview image (localizer). It is self-evident that the examination region can, alternatively or additionally, also be defined automatically, for example on the basis of a selected protocol.

The term "image", as used herein, means a data structure that represents a spatial distribution of a physical signal. The spatial distribution may be of any dimension, for example 2D, 3D, 4D or any higher dimension. The spatial distribution may be of any shape, for example forming a grid and thereby defining pixels, the grid being possibly irregular or regular. The physical signal is preferably caused by protons in the examination region. The physical signal can e.g. be an electrical current induced in a receiver coil by the precession of the net magnetization during resonance. The representation of the spatial distribution of the physical signal can e.g. be a color or level of gray, such that the image may be a 2D or 3D RGB/grayscale image.

A "synthetic contrast-enhanced image" is a representation of an examination region of an examination object.

The term "synthetic" means that the image is not the result of a (real) physical measurement, but of a calculated prediction. The synthetic contrast-enhanced image may, for example, show the examination region as it would appear as a result of an MR examination after a high-dose (e.g., full-dose or a dose higher than the standard dose) contrast agent had been applied - without this dose actually being applied. The synthetic contrast-enhanced image can be predicted on the basis of MR data, the MR data representing the examination region after the (real) application of a low-dose contrast agent.

In other words: MR data representing the examination region after the application of a first dose of a contrast agent are used to predict a synthetic contrast-enhanced image that represents the examination region after the application of a second dose of a contrast agent, wherein the second dose is different from the first dose, preferably higher than the first dose.

The prediction of the synthetic contrast-enhanced image is done, at least partially, on the basis of MR data. The MR data are a result of one or more (real) MR examinations of the examination object.

The MR data comprise a plurality of representations of the examination region. The term "plularity" means more than one, preferably more than 2, more preferably more than 3, even more preferably more than 4, most preferably more than 5. Usually, each of representations represents the examination region differently. In other words: in each representation, the examination region has a different appearance. It is for example possible, that the contrast distribution is different, and/or that the signal intensities of one or more tissue types are different. Additionally or alternatively, the distribution of the contrast agent within the examination region can be different. Additionally or alternatively, different MR data can represent different areas (portions) of the examination region, whereas the areas (portions) may partially overlap.

The MR data represent the examination region within the examination object after administration of a first dose of a contrast agent.

The first dose is preferably a dose which is equal to or less than the dose which is recommended by the manufacturer or distributor of the contrast agent. Preferably, the first dose is equal to or less than a standard dose approved by an authority (e.g. the US Food and Drug Administration (FDA) or the European Medicines Agency (EMA)) for an MR examination.

The MR data can be or comprise one or more images, i.e. spatial representation(s) of the examination region, the MR data can be or comprise k-space data representing the examination region in frequency space, and/or the MR data can be one or more other representations of the examination region.

The MR data were acquired according to an MR sequence protocol. An "MR sequence" is usually a number of radiofrequency pulses and gradients that result in one or more MR images (or any other MR representation(s)) with a particular appearance.

The MR sequence can be a T1-weighted sequence (T1w), a T2 weighted sequence (T2w), a volumetric interpolated breath-hold examination (VIBE) sequence, a fluid attenuated sequence (such as e.g. a fluid-attenuated inversion-recovery sequence (FLAIR)), a DIXON-type sequence, a diffusion-weighted imaging sequence (DWI), a proton density weighted sequence, a short TI inversion recovery sequence (STIR), and/or the like. Preferably, the MR sequence is a T1-weighted sequence, a T1-VIBE sequence, and/or a DIXON-type sequence.

In view of the fact that different MR sequences are available from different sources such as vendors of MR equipment, a skilled person can make use of further suitable MR sequences.

Such MR sequence can result in one or more MR images (or one or more other MR representations) which can be used for training a machine learning model to predict e.g. full-contrast images on the basis of low-contrast images. A T1-weighted DIXON-type sequence, for example, can generate 4 MR images (or other MR representations): an in-phase image (standard T1), an opposed phase image, a water-only image (fat suppressed T1), and a fat-only image.

In a preferred embodiment of the present disclosure, the MR data comprise an in-phase image, an opposed phase image, a water-only image, and a fat-only image, with all images acquired according to a DIXON-type sequence protocol, preferably according to a T1-weighted DIXON-type sequence protocol, more preferably according to a T1-VIBE-DIXON-type sequence protocol.

In the method described in WO2019/074938A1, a synthetic contrast-enhanced image (a so-called full-contrast image) representing an examination region is based on two (real) MR images: a low-contrast image of the examination region, and a zero-contrast image of the examination region.

Surprisingly, it has been found that synthetic contrast-enhanced images can be generated solely on the basis of one or more MR images (or other MR representations) acquired after administration of a contrast agent; a zero-contrast image (also referred to as pre-dose image or native image) is not required and can therefore be omitted.

Thus, in a preferred embodiment, no MR data representing the examination region without contrast agent are used for training and/or prediction. In other words, in a preferred embodiment, the present invention provides a method of training and/or using a machine learning model to generate a synthetic contrast-enhanced MR image that represents an examination region within an examination object without making use of MR data acquired prior to the administration of the first dose of the contrast agent.

Thus, a preferred embodiment of the present disclosure relates to a method of training a machine learning model to generate a synthetic contrast-enhanced MR image that represents at least a portion of an examination region within an examination object, the method comprising the steps of:
- receiving a training data set, the training data set comprising, for each object of a multitude of objects, MR data, and target MR data,
   - wherein the MR data represent the examination region within the object after administration of a first dose of a contrast agent,
   - wherein the target MR data represent at least a portion of the examination region within the object after administration of a second dose of a contrast agent, wherein the second dose is different from the first dose,
- training the machine learning model, thereby obtaining a trained machine learning mode, wherein the training comprises:
   ∘ inputting the MR data into the machine learning model,
   wherein the machine learning model is configured to generate, solely on the basis of the MR data, and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
- outputting the trained machine learning model, and/or storing the machine learning model on a data storage, and/or providing the trained machine learning model for predictive purposes.

The fact a zero-contrast representation of the examination region is not required means an acceleration of the examination procedure. In addition, the time a patient spends in the tomograph can be reduced.

Additionally, it has been found that the quality of synthetic contrast-enhanced MR images can be improved if the prediction is based on MR data representing the examination region within the object after administration of different doses of a contrast agent and/or after administration of different contrast agents.

Thus, another preferred embodiment of the present disclosure relates to a method of training a machine learning model to generate a synthetic contrast-enhanced MR image that represents at least a portion of an examination region within an examination object, the method comprising the steps of:
- receiving a training data set, the training data set comprising, for each object of a multitude of objects, first MR data, second MR data, and target MR data,
   - wherein the first MR data represent the examination region within the object after administration of a first dose of a first contrast agent,
   - wherein the second MR data represent the examination region within the object after the administration of a second dose of the first contrast agent, or after the administration of a first dose of a second contrast agent,
   - wherein the target MR data represent at least a portion of the examination region within the object after administration of a target dose of the first and/or the second contrast agent, wherein the target dose is different from the first dose and/or from the second dose,
- training the machine learning model, thereby obtaining a trained machine learning mode, wherein the training comprises:
   ∘ inputting the first MR data, and the second MR data into the machine learning model,
      wherein the machine learning model is configured to generate, at least partially on the basis of the first MR data, the second MR data, and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
   - outputting the trained machine learning model, and/or storing the machine learning model on a data storage, and/or providing the trained machine learning model for predictive purposes.

Preferably, the first dose is smaller than the second dose, and the second dose is smaller than the target dose.

Additionally, it has been found that the quality of synthetic contrast-enhanced MR images can be improved if the prediction is based on MR data generated according to different MR sequence protocols.

So, instead of using only one set of MR data acquired according to a certain MR sequence protocol, more than one sets of MR data are used for training and prediction purposes, each set of MR data being acquired according to a different MR sequence protocol. The number of sets of MR data is at least two (first MR data and second MR data). Preferably the number of sets of MR data is in the range of 2 to 10.

Thus, another preferred embodiment of the present disclosure relates to a method of training a machine learning model to generate a synthetic contrast-enhanced MR image that represents at least a portion of an examination region within an examination object, the method comprising the steps of:
- receiving a training data set, the training data set comprising, for each object of a multitude of objects, first MR data, second MR data, and target MR data,
   - wherein the first MR data represent the examination region within the object after administration of a first dose of a contrast agent, wherein the first MR data were acquired according to a first MR sequence protocol,
   - wherein, the second MR data represent the examination region within the object after the administration of the first dose of the contrast agent, wherein the second MR data were acquired according to a second MR sequence protocol, wherein the second MR sequence protocol differs from the first MR sequence protocol,
   - wherein the target MR data represent at least a portion of the examination region within the object after administration of a second dose of a contrast agent, wherein the second dose is different from the first dose,
- training the machine learning model, thereby obtaining a trained machine learning mode, wherein the training comprises:
   ∘ inputting the first MR data, and the second MR data into the machine learning model,
   wherein the machine learning model is configured to generate, at least partially on the basis of the first MR data, the second MR data, and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
- outputting the trained machine learning model, and/or storing the machine learning model on a data storage, and/or providing the trained machine learning model for predictive purposes.

The first MR data represent the examination region within an object after administration of a first dose of a contrast agent, wherein the first MR data were acquired according to a first MR sequence protocol.

The first MR data can be or comprise one or more images, i.e. spatial representation(s) of the examination region, the first MR data can be or comprise k-space data representing the examination region in frequency space, and/or the first MR data can be or comprise one or more other representations of the examination region, provided that changes in the first MR sequence affect the appearance of the examination region.

The examination region is preferably a slice within the examination object. The thickness and location of the slice within the examination object is usually determined by the first MR sequence protocol.

The first dose is preferably a dose which is equal to or less than the dose which is recommended by the manufacturer or distributor of the contrast agent. Preferably, the first dose is equal to or less than a standard dose approved by an authority (e.g. the US Food and Drug Administration (FDA) or the European Medicines Agency (EMA)) for an MR examination.

The first MR sequence protocol describes the first MR sequence that was executed (used) to generate the first MR data.

The second MR data represent the examination region within the object after the administration of the first dose of the contrast agent, wherein the second MR data were acquired according to a second MR sequence protocol, wherein the second MR sequence protocol differs from the first MR sequence protocol

The second MR data, like the first MR data, can be or comprise one or more images, i.e. spatial representation(s) of the examination region, the second MR data can be or comprise k-space data representing the examination region in frequency space, and/or the second MR data can be or comprise one or more other representations of the examination region, provided that changes in the second MR sequence affect the appearance of the examination region.

In a preferred embodiment, first and/or second MR data were acquired according to one or more of the following MR sequence protocols: T1-weighted sequence (T1w), T2 weighted sequence (T2w), volumetric interpolated breath-hold examination (VIBE) sequence, fluid attenuated sequence (such as e.g. a fluid-attenuated inversion-recovery sequence (FLAIR)), DIXON-type sequence, diffusion-weighted imaging sequence (DWI), proton density weighted sequence, short TI inversion recovery sequence (STIR), and/or the like.

Another preferred embodiment of the present disclosure relates to a method of training a machine learning model to generate a synthetic contrast-enhanced MR image that represents at least a portion of an examination region within an examination object, the method comprising the steps of:
- receiving a training data set, the training data set comprising, for each object of a multitude of objects, first MR data, second MR data, third MR data and target MR data,
   - wherein the first MR data represent the examination region within the object after administration of a first dose of a contrast agent, wherein the first MR data were acquired according to a first MR sequence protocol,
   - wherein, the second MR data represent the examination region within the object after the administration of the first dose of the contrast agent, wherein the second MR data were acquired according to a second MR sequence protocol, wherein the second MR sequence protocol differs from the first MR sequence protocol,
   - wherein, the third MR data represent the examination region within the object without contrast agent, wherein the third MR data were acquired according to the first MR sequence protocol, the second MR sequence protocol, or a third MR sequence protocol, wherein the third MR sequence protocol differs from the first MR sequence protocol and/or from the second MR sequence protocol,
   - wherein the target MR data represent at least a portion of the examination region within the object after administration of a second dose of a contrast agent, wherein the second dose is different from the first dose,
- training the machine learning model, thereby obtaining a trained machine learning mode, wherein the training comprises:
   ∘ inputting the first MR data, the second MR data, and the third MR data into the machine learning model,
      wherein the machine learning model is configured to generate, at least partially on the basis of the first MR data, the second MR data, the third MR data and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
- outputting the trained machine learning model, and/or storing the machine learning model on a data storage, and/or providing the trained machine learning model for predictive purposes.

Additionally, it has been surprisingly found that the quality of synthetic contrast-enhanced images improves when the prediction of a synthetic contrast-enhanced image of an examination region is not based only on MR data representing that examination region, but additionally on MR data representing one or more other examination regions, preferably one or more examination regions adjacent to that examination region (whereas two or more examination regions may overlap each other).

So, instead of using only MR data representing the examination region, one or more additional MR data are used, the additional MR data representing additional examination regions. The number of sets of MR data representing different examination regions is at least two (first MR data and second MR data). Preferably the number of sets of MR data is in the range of 2 to 500.

Two examination regions differ if they are not identical. This means that
- two (or more) examination regions can be separated from each other, or
- two (or more) examination regions can partially overlap, or
- one (or more) examination region(s) can be located within another examination region.

Thus, another preferred embodiment of the present disclosure relates to a method of training a machine learning model to generate a synthetic contrast-enhanced MR image that represents at least a portion of an examination region within an examination object, the method comprising the steps of:
- receiving a training data set, the training data set comprising, for each object of a multitude of objects, first MR data, second MR data, and target MR data,
   - wherein the first MR data represent a first examination region within the object after administration of a first dose of a contrast agent, wherein the first MR data were acquired according to a first MR sequence protocol,
   - wherein, the second MR data represent a second examination region within the object after the administration of the first dose of the contrast agent, wherein the second MR data were acquired according to the first MR sequence protocol or a second MR sequence protocol,
   - wherein the target MR data represent the first examination region within the object after administration of a second dose of a contrast agent, wherein the second dose is different from the first dose,
- training the machine learning model, thereby obtaining a trained machine learning mode, wherein the training comprises:
   ∘ inputting the first MR data, and the second MR data into the machine learning model,
      wherein the machine learning model is configured to generate, at least partially on the basis of the first MR data, the second MR data, and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
- outputting the trained machine learning model, and/or storing the machine learning model on a data storage, and/or providing the trained machine learning model for predictive purposes.

In a preferred embodiment, in addition to first MR data representing a first examination region, second MR data are used for training and prediction, the second MR data representing a second examination region that is adjacent to the first examination region. Preferably, the first examination region and the second examination region are plane slices that are arranged parallel to each other. The first examination region and the second examination region may partially overlap; preferably the degree of overlap is in the range of 0% to 50%.

In another preferred embodiment, in addition to first MR data representing a first examination region, second MR data and third MR data are used for training and prediction, the second MR data representing a second examination region and the third MR data representing a third examination region. Preferably, the first examination region lies between the second examination region and the third examination region. Preferably, the first examination region, the second examination region, and the third examination region are plane slices that are arranged parallel to each other. The first examination region and the second examination region may partially overlap; preferably the degree of overlap is in the range of 0% to 50%. The first examination region and the third examination region may partially overlap; preferably the degree of overlap is in the range of 0% to 50%.

The second MR data (and any further MR data such as third MR data, fourth MR data, ...) can be one or more images, i.e. spatial representation(s) of the second (or third, or fourth,..., respectively) examination region, the second MR data (and any further MR data such as third MR data, fourth MR data, ...) can be k-space data representing the second (or third, or fourth,..., respectively) examination region in frequency space, or the second MR data (and any further MR data such as third MR data, fourth MR data, ...) can be one or more other representation of the second (or third, or fourth,...) examination region.

In another preferred embodiment, in addition to first MR data representing a first examination region, second MR data are used for training and prediction, wherein the second MR data represent a number of additional examination regions within the object after the administration of the first dose of the contrast agent, wherein the additional examination regions together with the first examination region form a stack of adjacent and/or partially overlapping slices.

Additionally, it has been found that, in general, the quality of the images usually increases when the database used for training is increased. Thus, the embodiments described herein can be combined with each other.

For example, the quality of synthetic contrast-enhanced images can be further improved by using MR data that comprise more than one representation of the examination region, and MR data that were acquired according to different MR sequence protocols.

Thus, another preferred embodiment of the present disclosure relates to a method of training a machine learning model to generate a synthetic contrast-enhanced MR image that represents at least a portion of an examination region within an examination object, the method comprising the steps of:
- receiving a training data set, the training data set comprising, for each object of a multitude of objects, first MR data, second MR data, optionally third MR data, and target MR data,
   - wherein the first MR data represent a first examination region within the object after administration of a first dose of a contrast agent, wherein the first MR data were acquired according to a first MR sequence protocol, wherein the first MR data preferably comprise more than one representation of the first examination region,
   - wherein the second MR data represent a number of additional examination regions within the object after the administration of the first dose of the contrast agent, wherein the additional examination regions together with the first examination region form a stack of adjacent and/or partially overlapping slices, wherein the second MR data were acquired according to the first MR sequence protocol or a second MR sequence protocol, wherein the second MR data preferably comprise more than one representation of at least some of the additional examination regions,
   - wherein the third MR data, if present, represent the first examination region and/or the second examination region without a contrast agent, wherein the third MR data were acquired according to the first MR sequence protocol or the second MR sequence protocol or a third sequence protocol,
   - wherein the target MR data represent the first examination region within the object after administration of a second dose of a contrast agent, wherein the second dose is different from the first dose,
- training the machine learning model, thereby obtaining a trained machine learning mode, wherein the training comprises:
   ∘ inputting the first MR data, the second MR data, and optionally the third MR data into the machine learning model,
      wherein the machine learning model is configured to generate, at least partially on the basis of the first MR data, the second MR data, optionally the third MR data and model parameters, predicted MR data,
   ∘ receiving from the machine learning model the predicted MR data,
   ∘ computing a loss value, the loss value quantifying the deviations between the predicted MR data and the target MR data,
   ∘ modifying one or more of the model parameters to minimize the loss value,
- outputting the trained machine learning model, and/or storing the machine learning model on a data storage, and/or providing the trained machine learning model for predictive purposes.

In a particularly preferred embodiment, the first MR data comprise an in-phase MR image, an opposed phase MR image, a water-only MR image, and a fat-only MR image. Preferably, the second MR data comprise, for each examination region of the number of examination regions, an opposed phase MR image, a water-only MR image, and a fat-only MR image. Preferably, the number of additional examination regions is 2, 4, 6, 8, or 10. Preferably the third MR data comprise a water-only MR image. Preferably, the first sequence protocol is a T1-weigthed DIXON-type sequence protocol, more preferably a T1-VIBE-DIXON-type sequence protocol. Preferably, the second sequence protocol is a T1-weigthed DIXON-type sequence protocol, more preferably a T1-VIBE-DIXON-type sequence protocol. Preferably the third sequence protocol is a T1-weigthed DIXON-type sequence protocol, more preferably a T1-VIBE-DIXON-type sequence protocol.

The generation of synthetic contrast-enhanced images in accordance with the present disclosure makes use of a trained machine learning model.

Such a "machine learning model", as described herein, may be understood as a computer implemented data processing architecture. The machine learning model can receive input data and provide output data based on that input data and the machine learning model, in particular the parameters of the machine learning model. The machine learning model can learn a relation between input data and output data through training. In training, parameters of the machine learning model may be adjusted in order to provide a desired output for a given input.

The process of training a machine learning model involves providing a machine learning algorithm (that is the learning algorithm) with training data to learn from. The term "trained machine learning model" refers to the model artifact that is created by the training process. The training data must contain the correct answer, which is referred to as the target. The learning algorithm finds patterns in the training data that map input data to the target, and it outputs a trained machine learning model that captures these patterns.

In the training process, training data are inputted into the machine learning model and the machine learning model generates an output. The output is compared with the (known) target. Parameters of the machine learning model are modified in order to reduce the deviations between the output and the (known) target to a (defined) minimum.

In general, a loss function can be used for training to evaluate the machine learning model. For example, a loss function can include a metric of comparison of the output and the target. The loss function may be chosen in such a way that it rewards a wanted relation between output and target and/or penalizes an unwanted relation between an output and a target. Such a relation can be e.g. a similarity, or a dissimilarity, or another relation.

A loss function can be used to calculate a loss value for a given pair of output and target. The aim of the training process can be to modify (adjust) parameters of the machine learning model in order to reduce the loss value to a (defined) minimum.

A loss function may for example quantify the deviation between the output of the machine learning model for a given input and the target. If, for example, the output and the target are numbers, the loss function could be the difference between these numbers, or alternatively the absolute value of the difference. In this case, a high absolute value of the loss function can mean that a parameter of the model needs to undergo a strong change.

In the case of a scalar output, a loss function may be a difference metric such as an absolute value of a difference, a squared difference.

In the case of vector-valued outputs, for example, difference metrics between vectors such as the root mean square error, a cosine distance, a norm of the difference vector such as a Euclidean distance, a Chebyshev distance, an Lp-norm of a difference vector, a weighted norm or any other type of difference metric of two vectors can be chosen. These two vectors may for example be the desired output (target) and the actual output.

For training the machine learning model according to the present disclosure, a training data set is required, the training data set comprising, for each object of a multitude of objects, MR data (e.g. first MR data, second MR data and optionally further data such as e.g. third MR data, fourth MR data, and so on, as described herein), and target MR data.

The term "multitude" as it is used herein means an integer greater than 1, usually greater than 10, preferably greater than 100.

It should be mentioned that the data set can be enlarged by augmentation techniques (see e.g. J. Nalepa et al.: Data Augmentation for Brain-Tumor Segmentation: A Review, Front. Comput. Neurosci., 11 December 2019 | https://doi.org/10.3389/fncom.2019.00083.

The target MR data represent the (first) examination region within the object after administration of a second dose of a contrast agent, wherein the second dose is preferably higher than the first dose.

Preferably, the target MR data were acquired according to the first MR sequence protocol.

Preferably, the first dose is a percentage in the range of 1% to 95% of the second dose, more preferably in the range of 5 % to 90 %, even more preferably in the range of 10% to 85%, most preferably in the range of 10% to 80%.

The first dose is preferably less than 50% of the second dose. As an example, in MRI contrast agents such as gadolinium chelates like gadopentetate dimeglumine (trade name: Magnevist^{®} and others), gadobenate dimeglumine (trade name: Multihance^{®}), gadoteric acid (Dotarem^{®}, Dotagita^{®}, Cyclolux^{®}), gadodiamide (Omniscan^{®}), gadoteridol (ProHance^{®}) and gadobutrol (Gadovist^{®}) are used. The contrast-enhancing effect of Primovist^{®}/Eovist^{®} is mediated by the stable gadolinium complex Gd-EOB-DTPA (gadolinium ethoxybenzyldiethylenetriaminepentaacetic acid). Primovist^{®} is injected intravenously as a bolus, in a weight-adapted dose. The recommended dose (the second dose) is 0.025 mmol/kg body weight or 0.1 ml/kg body weight. In the context of the present disclosure, a dose corresponding to 100% of the second dose of the contrast agent signifies, for example in connection with Gd-EOB-DTPA disodium, that 0.025 mmol/kg body weight or 0.1 ml/kg body weight is administered to the examination object. Accordingly, "preferably less than 50% of the second dose" signifies that at most 0.0125 mmol/kg body weight or at most 0.05 ml/kg body weight is administered to the examination object.

In one preferred embodiment, the second dose is the dose which is recommended by the manufacturer or distributor of the contrast agent. The recommended dose is usually the dose which is mentioned in the product label of the contrast agent and the dose approved by an authority (e.g. the US Food and Drug Administration (FDA) or the European Medicines Agency (EMA)) for an MR examination (also referred to as standard dose).

However, the second dose may also be higher than the standard dose. Thus, in another preferred embodiment, the second dose is a dose that is required to obtain a defined appearance (e.g. a defined contrast enhancement) of the (first) examination region, whereas the first dose is preferably equal to or less than the standard dose.

During training, MR data (e.g. the first MR data and the second MR data and optionally further data) are inputted into the machine learning model. The machine learning model is configured to generate, at least partially on the basis of the inputted MR data, and on the basis of model parameters, predicted MR data.

The predicted MR data are compared with the target MR data. A loss value using a loss function can be computed, the loss value quantifying the deviations between the predicted MR data and the target MR data.

During training, model parameters are modified in order to reduce the loss values to a defined minimum.

Once the pre-defined minimum is reached and/or the predictions generated by machine learning model meet pre-defined criteria, the training can be finished.

A validation data set can be used to determine the accuracy of the trained machine learning model.

The trained machine learning model can be outputted and/or stored in a data storage and/or used for predictive purposes.

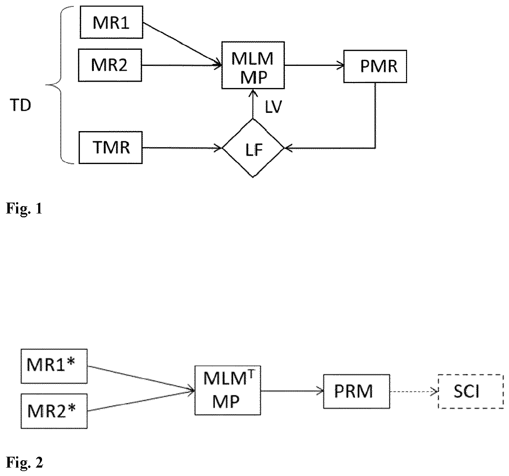

Fig. 1 shows schematically by way of example, the training of a machine learning model. The training is based on a training data set TD. The training data set TD comprises, for each examination object of a multitude of examination objects, first MR data MR1, second MR data MR2 and target MR data TMR. In the example shown in Fig. 1, only one data set comprising first MR data MR1, second MR data MR2 and target MR data TMR is shown. First MR data MR1 and second MR data MR2 are inputted into the machine learning model MLM. The machine learning model MLM is configured to generate, at least partially on the basis of the inputted data and model parameters MP, predicted MR data PMR. The predicted MR data PMR are compared with the target MR data TMR. This is done by using a loss function LF, the loss function quantifying the deviations between the predicted MR data PMR and the target MR data TMR. For each pair of predicted MR data and the respective target MR data, a loss value LV is computed. During training the model parameters MP are modified in a way that reduces the loss values to a defined minimum. The aim of the training is to let the machine learning model MLM generate for each set of first MR data and second MR data predicted MR data which come as close to the corresponding target MR data as possible. Once the defined minimum is reached, the (now fully trained) machine learning model can be used to predict an output for new input data (input data which have not been used during training and for which the target is usually not (yet) known).

Fig. 2 shows schematically by way of example how a trained machine learning model can be used for making predictions. The trained machine learning model MLM^{T} can be the machine learning model described with reference to Fig. 1. New input data comprising new first MR data MR1^{∗} and new second MR data MR2^{∗} are inputted into the trained machine learning model MLM^{T}. The trained machine learning model MLM^{T} is configured and trained to generate, at least partially on the basis of the new input data and the model parameters MD, predicted MR data PRM

The predicted MR data represents the first examination region after application of a second dose of a contrast agent, the second dose being different from (preferably higher than) the first dose.

Depending on the nature of the input data (first MR data, second MR data, optionally further input data), the predicted MR data can be a spatial representation of the first examination region, or a representation of the first examination region in frequency space, or another representation of the first examination region. If for example, the input data comprise one or more images (i.e. spatial representations) of the first examination region, then the predicted MR data preferably (but not necessarily) constitute an image of the first examination region, too. If for example, the input data comprise one or more k-space data sets or any other representation of the first examination region, then the predicted MR data preferably (but not necessarily) constitute k-space data (or the other representation) of the first examination, too.

In case the predicted MR data do not constitute an image (i.e. a spatial representation) or do not comprise an image, a further step may be necessary to transform the predicted MR data into an image; e.g. an inverse Fourier-transformation may be required to transform k-space data into an image. This is reflected in Fig. 2 by the dashed arrow which indicates one or more optional transformation steps to transform the predicted MR data PRM into one or more synthetic contrast-enhanced image(s) SCI.

It should be noted that using representations of examination regions in frequency space may have advantages over using representations of examination regions in real space.

One advantage of the use of representations in frequency space of the examination region may be that contrast information is separated from detail information (e.g., fine structures). It is thus possible to concentrate, during a training procedure, on the contrast information to be learnt by the predictive machine learning model and to also concentrate, during a prediction procedure, on the contrast information that may be predicted by the trained machine learning model. Whereas contrast information in a representation in real space of an examination region is usually distributed over an entire representation (e.g., each image element intrinsically bears information about contrast), the contrast information in a representation in frequency space of an examination region is encoded in and around the center of the frequency space. Accordingly, the low frequencies in a representation in frequency space of an examination region are responsible for the contrast information, whereas the high frequencies contain detail information about fine structures. Using of representations in frequency space of the examination region may make it possible to separate the contrast information, to limit training and prediction to the contrast information, and to re-introduce information about the fine structures after a training procedure and/or a prediction procedure.

Further, representations in frequency space of the examination region may be more tolerant with respect to errors in co-registration during training, validation, and prediction procedures than representations in real space of the examination region. For example, if a representation in frequency space is superimposed with another representation in frequency space with less accuracy, the lack of accuracy has less influence than if representations in real space of the examination region are superimposed with less accuracy.

Thus, in a preferred embodiment of the present disclosure, MR data (e.g. first MR data, second MR data, and any further MR data used for training and prediction purposes), target MR data and predicted MR data are representation in frequency space of examination region(s).

The machine learning model according to the present disclosure can be or comprise one or more artificial neural networks.

An artificial neural network (ANN) is a biologically inspired computational model. An ANN usually comprises at least three layers of processing elements: a first layer with input neurons (nodes), a *k*th layer with at least one output neuron (node), and *k*-2 inner (hidden) layers, where *k* is an integer greater than 2.

In such a network, the input neurons serve to receive the input data. If the input data constitute or comprise an image (e.g. a spectrogram representation), there is usually one input neuron for each pixel/voxel of the input image; there can be additional input neurons for additional input data such as data about measurement conditions, data about the subject which generated the body action and/or the like. The output neurons serve to output the output data (result(s)).

The processing elements of the layers are interconnected in a predetermined pattern with predetermined connection weights therebetween. Each network node usually represents a (simple) calculation of the weighted sum of inputs from prior nodes and a non-linear output function. The combined calculation of the network nodes relates the inputs to the outputs.

When trained, the connection weights between the processing elements in the ANN contain information regarding the relationship between the input data and the output data which can be used to predict new output data from a new input data.

Each network node represents a calculation of the weighted sum of inputs from prior nodes and a non-linear output function. The combined calculation of the network nodes relates the inputs to the output(s).

Separate networks can be developed for each property measurement or groups of properties can be included in a single network. Preferably, different dimensions and/or modalities of patient data (and/or optionally additional data) are combined in a joint representation.

Training estimates network weights that allow the network to calculate (an) output value(s) close to the measured output value(s). A supervised training method can be used in which the output data is used to direct the training of the network weights. The network weights can be initialized with small random values or with the weights of a prior partially trained network. The training data inputs are applied to the network and the output values are calculated for each training sample. The network output values are compared to the measured output values. A backpropagation algorithm can be applied to correct the weight values in directions that reduce the error between measured and calculated outputs. The process is iterated until no further reduction in error can be made or until a predefined prediction accuracy has been reached.

A cross-validation method can be employed to split the data into training and validation data sets. The training data set is used in the backpropagation training of the network weights. The validation data set is used to verify that the trained network generalizes to make good predictions. The best network weight set can be taken as the one that best predicts the outputs of the training data. Similarly, varying the number of network hidden nodes and determining the network that performs best with the data sets optimizes the number of hidden nodes.

In a preferred embodiment of the present invention, the machine learning model is or comprises one or more convolutional neural networks (CNN).

A CNN is a class of deep neural networks, most commonly applied to analyzing visual imagery (such as spectrogram representations). A CNN comprises an input layer with input neurons, an output layer with at least one output neuron, as well as multiple hidden layers between the input layer and the output layer.

The hidden layers of a CNN typically consist of convolutional layers, ReLU (Rectified Linear Units) layer i.e. activation function, pooling layers, fully connected layers and normalization layers.

The nodes in the CNN input layer are organized into a set of "filters" (feature detectors), and the output of each set of filters is propagated to nodes in successive layers of the network. The computations for a CNN include applying the convolution mathematical operation to each filter to produce the output of that filter. Convolution is a specialized kind of mathematical operation performed by two functions to produce a third function that is a modified version of one of the two original functions. In convolutional network terminology, the first function to the convolution can be referred to as the input, while the second function can be referred to as the convolution kernel. The output may be referred to as the feature map. For example, the input to a convolution layer can be a multidimensional array of data that defines the various color components or grey scale values of an input image. The convolution kernel can be a multidimensional array of parameters, where the parameters are adapted by the training process for the neural network. More details about how to implement a convolutional neural network can be found in the literature (see e.g. Yu Han Liu: Feature Extraction and Image Recognition with Convolutional Neural Networks, 2018, J. Phys.: Conf. Ser. 1087 062032; H. H. Aghdam et al.: Guide to Convolutional Neural Networks, Springer 2017, ISBN: 978-3-319-57549-0; S. Khan et al.: Convolutional Neural Networks for Computer Vision, Morgan & Claypool Publishers, 2018, ISBN: 978-1-681-730219).

In a preferred embodiment of the present disclosure, the machine learning model is based on a specific kind of convolutional architecture called U-Net (see e.g. O. Ronneberger et al.: U-net: Convolutional networks for biomedical image segmentation, in: International Conference on Medical image computing and computer-assisted intervention, pp. 234-241, Springer, 2015, https://doi.org/10.1007/978-3-319-24574-4_28). The U-Net architecture consists of two main blocks, an encoding path and a decoding path. The encoding path uses convolutions, activation functions and pooling layers to extract image features, while the decoding path replaces the pooling layers with upsampling layers to project the extracted features back to pixel space, and finally recovers the image dimension at the end of the architecture.

These are used in combination with activation functions and convolutions. Finally, the feature maps from the encoding paths can be concatenated to the feature maps in the decoding path in order to preserve fine details from the input data.

In another preferred embodiment, the machine learning model is or comprises a generative adversarial network (GAN), preferably a Pix2Pix GAN or a cycleGAN. A GAN is a class of machine learning frameworks in which two artificial neural networks contest with each other. A first neural networks is configured to generate a synthetic contrast-enhanced image, a second network is configured to distinguish synthetic contrast-enhanced images from real images. Both networks are trained simultaneously: the first network is trained to generate better synthetic images which the second network is not able to distinguish from real images, the second network is trained to distinguish increasingly good synthetic images from real images. It should be noted that the competing networks can be trained not only with images but also with other representations (such as k-space data). It should be noted that artificial neural networks intended for unpaired image-to-image translations can also be used. Details about GANs can be found e.g. in M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887. The network should preferably be adapted to the image depth and resolution of the MR data because many networks only work with 8bit and 256x256 pixels resolution. A particularly preferred machine learning model is disclosed by J. Haubold et al.: Contrast agent dose reduction in computed tomography with deep learning using a conditional generative adversarial network, Eur Radiol. 2021, 31(8): 6087-6095, doi: 10.1007/s00330-021-07714-2.

The loss value is computed on the basis of the predicted MR data and the target MR data using a loss function. Examples of loss functions that can be used include L1 loss, L2 loss, structure similarity index measure (SSIM) or combination of the above to name a few. More details about loss functions may be found in the scientific literature (see e.g.: K. Janocha et al.: On Loss Functions for Deep Neural Networks in Classification, 2017, arXiv:1702.05659v1 [cs.LG]; H. Zhao et al.: Loss Functions for Image Restoration with Neural Networks, 2018, arXiv:1511.08861v3 [cs.CV]).

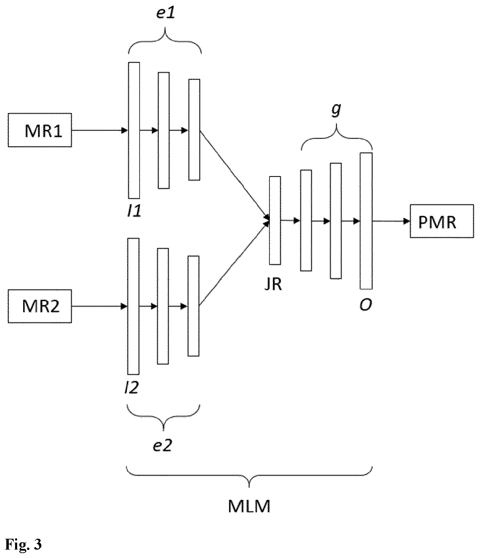

Fig. 3 shows schematically, by way of example, a preferred embodiment of the machine learning model. The machine learning model comprises an artificial neural network comprising three subnetworks. A first subnetwork *e1* is configured to receive first MR data MR1 via a first input layer *I1*. A second subnetwork *e2* is configured to receive second MR data MR2 via a second input layer 12. Both subnetworks *e1* and *e2* are configured to generate, at least partially on the basis of the inputted data, compressed representations of the inputted data.

The term "compressed" means that the compressed representation is of lower dimensionality than the inputted data. In other words: the machine learning model is trained to reduce the dimensionality of the inputted data and thereby focusing on features which are relevant for the prediction task (predicted MR data generation task).

A compressed representation of first MR data MR1 and a compresses representation of second MR data MR2 are then concatenated to a joint representation JR. The term "concatenation" as it is used herein means the combination of two or more pieces of data into a single piece of data. For example, two vectors may be concatenated to a single vector.

When two or more originally disjoint pieces of data are combined to one piece of data, they form a joint representation. Such a joint representation may comprise (e.g., contain) essentially e.g. all or most of the information comprised (e.g., contained) in the individual pieces of data that the joint representation was derived of. The joint representation may also comprise (e.g., contain) a part of the information included in both or one of the individual pieces of data.

Combination(s) of (e.g. at least two) representations to generate a joint representation may in case of vectors and/or matrices and/or tensors for example be achieved by a multiplication, an addition, for example an element-wise addition, a cross-product, a stacking on top of each other, and many other procedures.

A third subnetwork g is configured to generate, at least partially on the basis of the joint representation JR, predicted MR data PMR. The predicted MR data PMR are outputted via the output O.

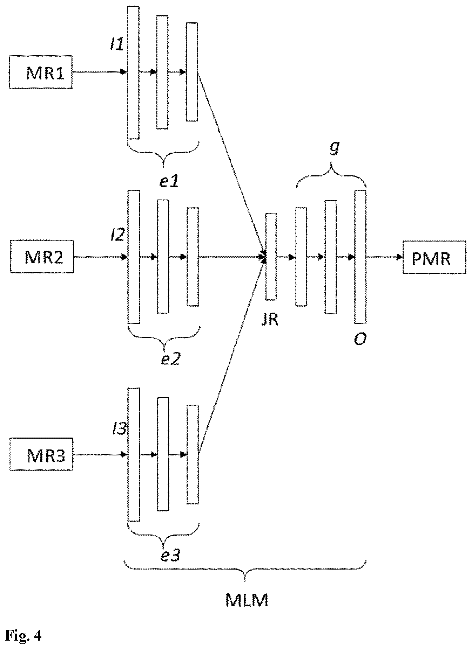

Fig. 4 shows schematically, by way of example, another preferred embodiment of the machine learning model. The machine learning model MLM is configured to generate, at least partially on the basis first MR data MR1, second MR data MR2, and third MR data MR3, predicted MR data PMR. First MR data MR1, second MR data MR2, and third MR data MR3 are inputted into the machine learning model MLM separately: first MR data MR1 into a first input *I1*, second MR data MR2 into a second input 12, and third MR data MR3 into a third input 13. After a compressed representation has been generated from each of the inputted data by means of three subnetworks *e1, e2,* and *e3,* a joint representation JR is generated from the compressed representations. A further subnetwork g is configured to generate, at least partially on the basis of the joint representation JR, predicted MR data PMR. The predicted MR data PMR are outputted via the output O.

The operations in accordance with the teachings herein may be performed by at least one computer specially constructed for the desired purposes or general-purpose computer specially configured for the desired purpose by at least one computer program stored in a typically non-transitory computer readable storage medium.

The term "non-transitory" is used herein to exclude transitory, propagating signals or waves, but to otherwise include any volatile or non-volatile computer memory technology suitable to the application.

The term "computer" should be broadly construed to cover any kind of electronic device with data processing capabilities, including, by way of non-limiting example, personal computers, servers, embedded cores, computing system, communication devices, processors (e.g. digital signal processor (DSP), microcontrollers, field programmable gate array (FPGA), application specific integrated circuit (ASIC), etc.) and other electronic computing devices.

The term "process" as used herein is intended to include any type of computation or manipulation or transformation of data represented as physical, e.g. electronic, phenomena which may occur or reside e.g. within registers and/or memories of at least one computer or processor. The term processor includes a single processing unit or a plurality of distributed or remote such units.

Any suitable input device, such as but not limited to a keyboard, a mouse, a microphone and/or a camera sensor, may be used to generate or otherwise provide information received by the system and methods shown and described herein. Any suitable output device or display, such as but not limited to a computer screen (monitor) and/or printer may be used to display or output information generated by the system and methods shown and described herein. Any suitable processor/s, such as bot not limited to a CPU, DSP, FPGA and/or ASIC, may be employed to compute or generate information as described herein and/or to perform functionalities described herein. Any suitable computerized data storage, such as but not limited to optical disks, CDROMs, DVDs, BluRays, magnetic-optical discs or other discs; RAMs, ROMs, EPROMs, EEPROMs, magnetic or optical or other cards, may be used to store information received by or generated by the systems shown and described herein. Functionalities shown and described herein may be divided between a server computer and a plurality of client computers. These or any other computerized components shown and described herein may communicate between themselves via a suitable computer network.

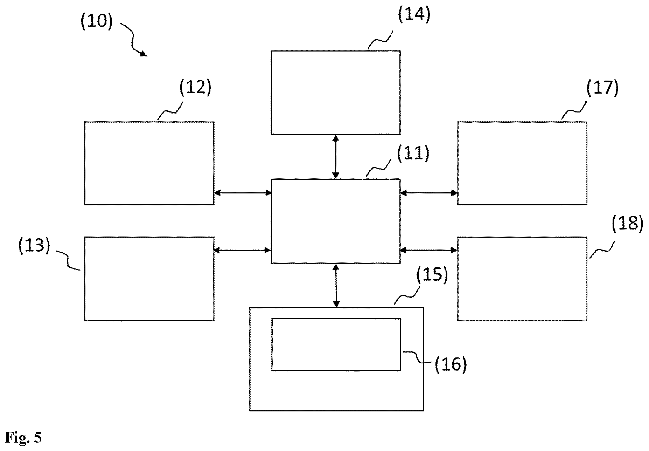

Fig. 5 illustrates a computer system (10) according to some example implementations of the present invention in more detail. Generally, a computer system of exemplary implementations of the present disclosure may be referred to as a computer and may comprise, include, or be embodied in one or more fixed or portable electronic devices. The computer may include one or more of each of a number of components such as, for example, processing unit (11) connected to a memory (15) (e.g., storage device).

The processing unit (11) may be composed of one or more processors alone or in combination with one or more memories. The processing unit is generally any piece of computer hardware that is capable of processing information such as, for example, data (incl. digital images), computer programs and/or other suitable electronic information. The processing unit is composed of a collection of electronic circuits some of which may be packaged as an integrated circuit or multiple interconnected integrated circuits (an integrated circuit at times more commonly referred to as a "chip"). The processing unit (11) may be configured to execute computer programs, which may be stored onboard the processing unit or otherwise stored in the memory (15) of the same or another computer.

The processing unit (11) may be a number of processors, a multi-core processor or some other type of processor, depending on the particular implementation. Further, the processing unit may be implemented using a number of heterogeneous processor systems in which a main processor is present with one or more secondary processors on a single chip. As another illustrative example, the processing unit may be a symmetric multi-processor system containing multiple processors of the same type. In yet another example, the processing unit may be embodied as or otherwise include one or more ASICs, FPGAs or the like. Thus, although the processing unit may be capable of executing a computer program to perform one or more functions, the processing unit of various examples may be capable of performing one or more functions without the aid of a computer program. In either instance, the processing unit may be appropriately programmed to perform functions or operations according to example implementations of the present disclosure.

The memory (15) is generally any piece of computer hardware that is capable of storing information such as, for example, data, computer programs (e.g., computer-readable program code (16)) and/or other suitable information either on a temporary basis and/or a permanent basis. The memory may include volatile and/or non-volatile memory, and may be fixed or removable. Examples of suitable memory include random access memory (RAM), read-only memory (ROM), a hard drive, a flash memory, a thumb drive, a removable computer diskette, an optical disk, a magnetic tape or some combination of the above. Optical disks may include compact disk - read only memory (CD-ROM), compact disk - read/write (CD-R/W), DVD, Blu-ray disk or the like. In various instances, the memory may be referred to as a computer-readable storage medium. The computer-readable storage medium is a non-transitory device capable of storing information, and is distinguishable from computer-readable transmission media such as electronic transitory signals capable of carrying information from one location to another.

Computer-readable medium as described herein may generally refer to a computer-readable storage medium or computer-readable transmission medium.

In addition to the memory (15), the processing unit (11) may also be connected to one or more interfaces (12, 13, 14, 17, 18) for displaying, transmitting and/or receiving information. The interfaces may include one or more communications interfaces (17, 18) and/or one or more user interfaces (12, 13, 14). The communications interface(s) may be configured to transmit and/or receive information, such as to and/or from other computer(s), network(s), database(s) or the like. The communications interface may be configured to transmit and/or receive information by physical (wired) and/or wireless communications links. The communications interface(s) may include interface(s) to connect to a network, such as using technologies such as cellular telephone, Wi-Fi, satellite, cable, digital subscriber line (DSL), fiber optics and the like. In some examples, the communications interface(s) may include one or more short-range communications interfaces configured to connect devices using short-range communications technologies such as NFC, RFID, Bluetooth, Bluetooth LE, ZigBee, infrared (e.g., IrDA) or the like.

The user interfaces (12, 13, 14) may include a display (14). The display (14) may be configured to present or otherwise display information to a user, suitable examples of which include a liquid crystal display (LCD), light-emitting diode display (LED), plasma display panel (PDP) or the like. The user input interface(s) (12, 13) may be wired or wireless, and may be configured to receive information from a user into the computer system (10), such as for processing, storage and/or display. Suitable examples of user input interfaces include a microphone, image or video capture device, keyboard or keypad, joystick, touch-sensitive surface (separate from or integrated into a touchscreen) or the like. In some examples, the user interfaces may include automatic identification and data capture (AIDC) technology for machine-readable information. This may include barcode, radio frequency identification (RFID), magnetic stripes, optical character recognition (OCR), integrated circuit card (ICC), and the like. The user interfaces may further include one or more interfaces for communicating with peripherals such as printers and the like.

As indicated above, program code instructions may be stored in memory, and executed by processing unit that is thereby programmed, to implement functions of the systems, subsystems, tools and their respective elements described herein. As will be appreciated, any suitable program code instructions may be loaded onto a computer or other programmable apparatus from a computer-readable storage medium to produce a particular machine, such that the particular machine becomes a means for implementing the functions specified herein. These program code instructions may also be stored in a computer-readable storage medium that can direct a computer, processing unit or other programmable apparatus to function in a particular manner to thereby generate a particular machine or particular article of manufacture. The program code instructions may be retrieved from a computer-readable storage medium and loaded into a computer, processing unit or other programmable apparatus to configure the computer, processing unit or other programmable apparatus to execute operations to be performed on or by the computer, processing unit or other programmable apparatus.

Retrieval, loading and execution of the program code instructions may be performed sequentially such that one instruction is retrieved, loaded and executed at a time. In some example implementations, retrieval, loading and/or execution may be performed in parallel such that multiple instructions are retrieved, loaded, and/or executed together. Execution of the program code instructions may produce a computer-implemented process such that the instructions executed by the computer, processing circuitry or other programmable apparatus provide operations for implementing functions described herein.

### Example

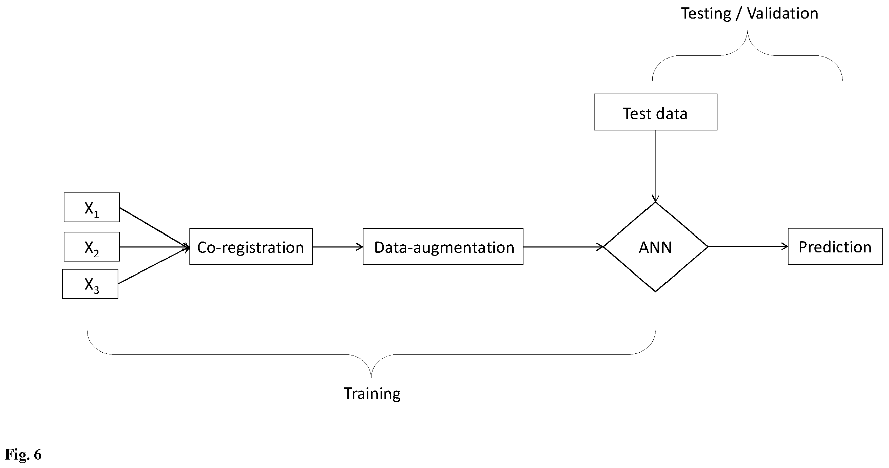

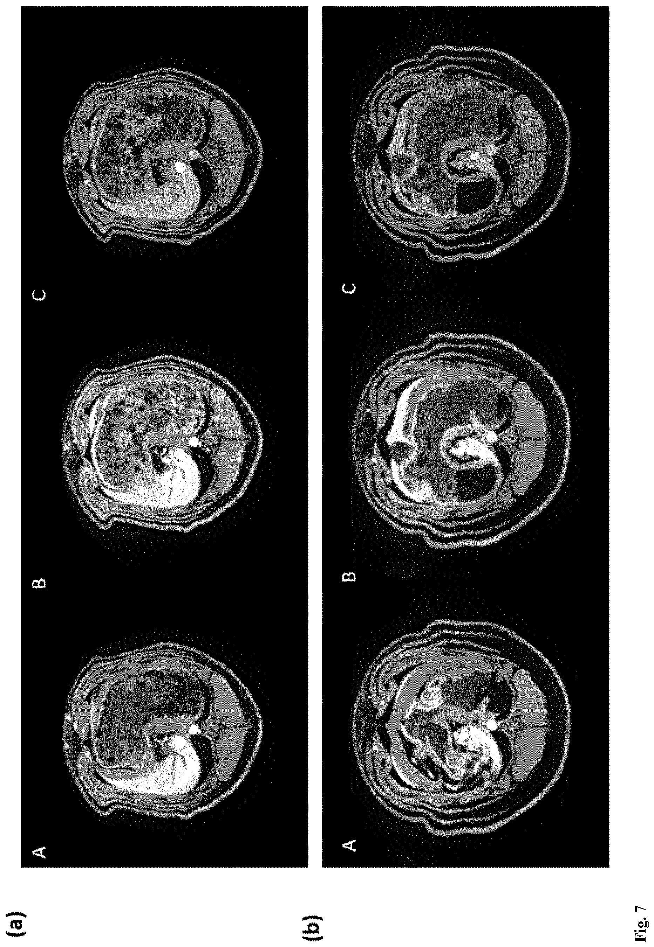

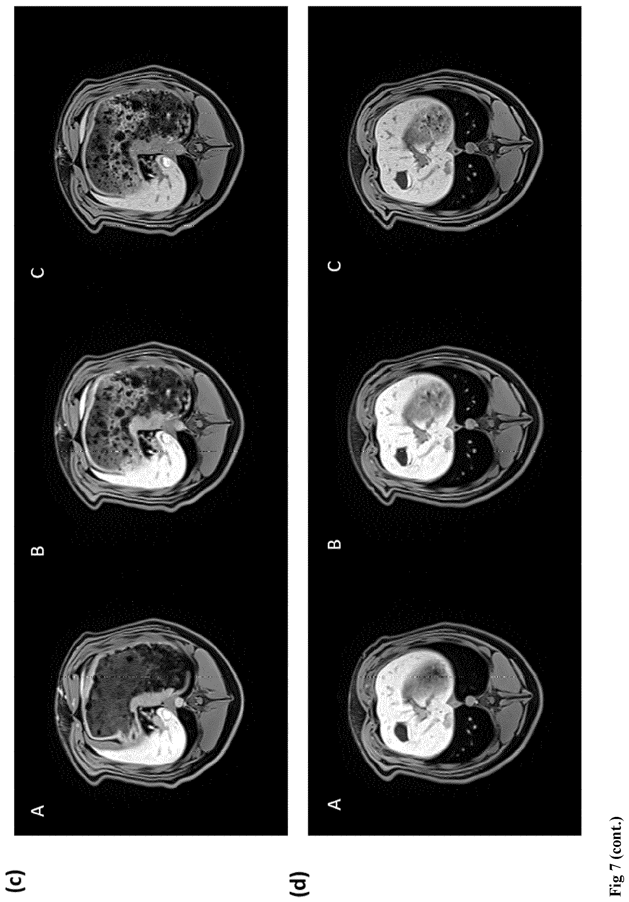

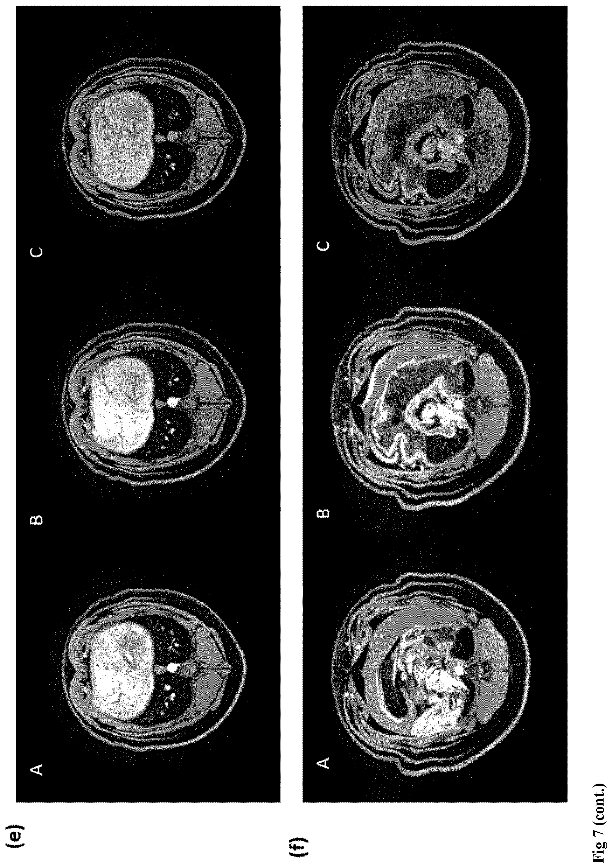

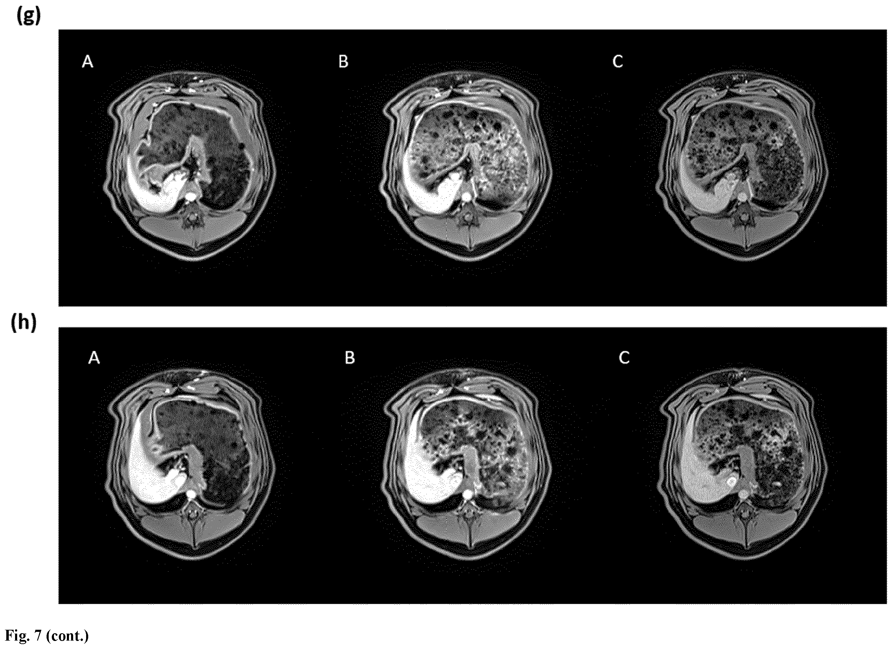

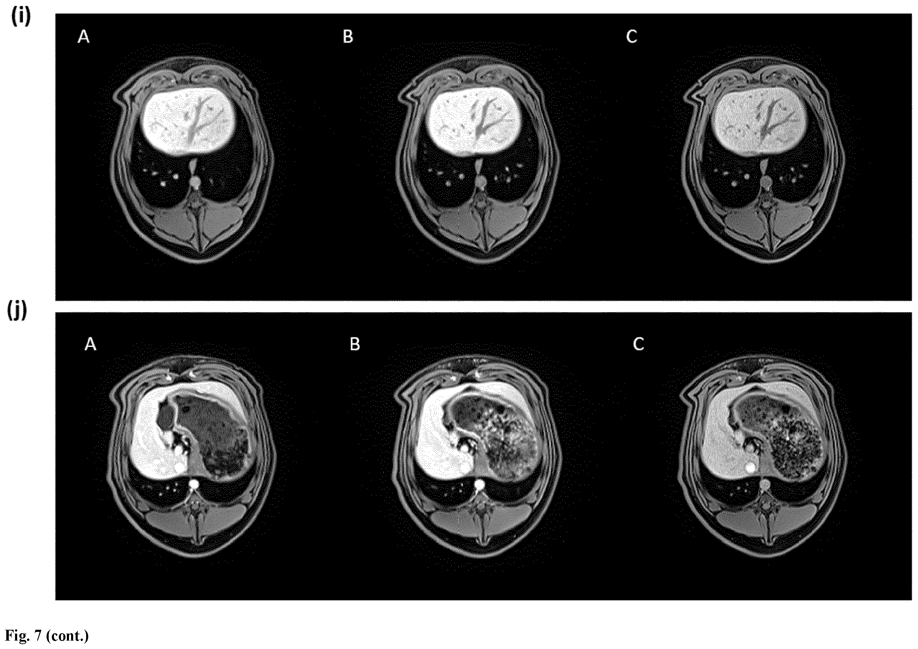

In the following, an example implementation to reduce the required contrast media dose by 80% in dynamic liver MRI with hepatobiliary contrast media is described. The description is based in Figs. 6 and 7. In the example implementation, to convert a slice X₂ to predict target Y, 15 MR images were used as input to the machine learning model. Comprising three stacks of five MR images each, the slice X₂, the slice above X₁ and the slice below X₃. Each of the image stacks contain the water map, the fat map, the in-phase and opposed phase of a T1-VIBE-DIXON acquired after injection of a contrast media dose reduced by 80% (0.005 mmol/kg, Gadoxetate) and a water map of a T1-VIBE-DIXON acquired before injection. The target Y to be predicted is located at the same position as slice X₂, containing the water map of a T1-VIBE-DIXON acquired with the standard dose (0.025 mmol/kg, Gadoxetate). The structure of the example implementation is shown in Fig. 6 and the predicted results of the machine learning mode are shown in Fig. 7 (a) to (j). In Fig. 7, 'A' shows the examination area after administration of a standard dose, 'C' shows the examination area after administration of a low dose, and 'B' shows the predicted images (contrast enhanced low dose). The machine learning model used is a GAN, more specifically a CycleGAN with the following parameters: Loss: Cycle consistency Loss; Upsampling: Transpose; Batch Size: 4; lr: 0.0002; lr_decay: linear; Epochs: 100 without decay + 100 with decay; net_generator: resnet9 _blocks; net_discriminator: 70x70 PatchGan; normalization_type: instance normalization. To acquire a data set, 20 healthy Goettingen minipigs underwent MRI on three separate occasions at reduced (0.005mmol/kg, Gadoxetate) and standard doses (0.025mmol/kg, Gadoxetate) containing an arterial, portal venous, venous, 20min and 30min hepatobiliary contrast phases. Three of 19 animals were randomly selected and withheld for validation (18 examinations). One animal had to be excluded because of incomplete examinations. The remaining 16 animals (96 examinations) were used to train the GAN for an image-to-image conversion. Subsequently, the data sets were co-registered and a data-augmentation was performed with the following parameters: resize: 224; crop size: 224; rotate: -15, 15; horizontal flipping. To validate the networks efficiency, ROI measurements were performed in the abdominal aorta, inferior vena cava, portal vein, liver parenchyma, and autochthonous back muscles, and the contrast to noise ratio (CNR) was calculated. Additionally, in a visual-Turing-test, the T1-VIBE-DIXON with the standard dose and the contrast enhanced low dose T1-VIBE-DIXON were demonstrated to three consultant radiologists, who had to decide whether they would have reported both data sets identical and which images originates from the standard dose T1-VIBE-DIXON. The CNR increased significantly(P<0.0001) in all contrast phases and was not significantly different from the standard dose examination (CNR: 19.8±6.2 low dose, 34.6±9.8 contrast enhanced low dose, 38.5±12.8 standard dose). In the VTT, the testers reported on average that, the standard dose and contrast enhanced low dose sequences were the same in 96% of the examinations. The testers could identify the standard dose sequence as such in 61% of the cases.

## Claims

1. A method comprising the steps:
- receiving a training data set, the training data set comprising, for each object of a multitude of objects, MR data, and target MR data,
- wherein the MR data comprise a plurality of representations of a first examination region within the object after administration of a first dose of a contrast agent,
- wherein the target MR data represent at least a portion of the first examination region within the object after administration of a second dose of a contrast agent, wherein the second dose is different from the first dose,
- training the machine learning model, thereby obtaining a trained machine learning mode, wherein the training comprises:
∘ inputting the MR data into the machine learning model,
wherein the machine learning model is configured to generate, at least partially on the basis of the MR data and model parameters, predicted MR data,
∘ receiving from the machine learning model the predicted MR data,
∘ computing a loss value, the loss value quantifying the deviations between the predicted MR data and the target MR data,
∘ modifying one or more of the model parameters to minimize the loss value,
- outputting the trained machine learning model, and/or storing the machine learning model on a data storage, and/or providing the trained machine learning model for predictive purposes.

2. The method according to claim 1, wherein the MR data comprise an in-phase image, an opposed phase image, a water-only image, and a fat-only image, with some or all images acquired according to a DIXON-type sequence protocol, preferably according to a T1-weighted DIXON-type sequence protocol, more preferably according to a T1-VIBE-DIXON-type sequence protocol.

3. The method according to claim 1 or 2, wherein the machine learning model is configured to generate the predicted MR data without making use of MR data acquired prior to the administration of the first dose of the contrast agent.

4. The method according to any one of claims 1 to 3, wherein the MR data comprise, for each object of the plurality of objects, first MR data and second MR data,
- wherein the first MR data represent the examination region within the object after administration of the first dose of the contrast agent, wherein the first MR data were acquired according to a first MR sequence protocol,
- wherein, the second MR data represent the examination region within the object after the administration of the first dose of the contrast agent, wherein the second MR data were acquired according to a second MR sequence protocol, wherein the second MR sequence protocol differs from the first MR sequence protocol,
- wherein the training of the machine learning model comprises:
∘ inputting the first MR data, and the second MR data into the machine learning model, wherein the machine learning model is configured to generate, at least partially on the basis of the first MR data, the second MR data, and model parameters, predicted MR data.

5. The method according to any one of claims 1 or 2, wherein the MR data comprise, for each object of the plurality of objects, first MR data, second MR data, and third MR data,
- wherein the first MR data represent the examination region within the object after administration of the first dose of the contrast agent, wherein the first MR data were acquired according to a first MR sequence protocol,
- wherein, the second MR data represent the examination region within the object after the administration of the first dose of the contrast agent, wherein the second MR data were acquired according to a second MR sequence protocol, wherein the second MR sequence protocol differs from the first MR sequence protocol,
- wherein, the third MR data represent the examination region within the object without contrast agent, wherein the third MR data were acquired according to the first MR sequence protocol, the second MR sequence protocol, or a third MR sequence protocol, wherein the third MR sequence protocol differs from the first MR sequence protocol and/or from the second MR sequence protocol,
- wherein the training of the machine learning model comprises:
∘ inputting the first MR data, the second MR data, and the third MR data into the machine learning model,
wherein the machine learning model is configured to generate, at least partially on the basis of the first MR data, the second MR data, the third MR data and model parameters, predicted MR data.

6. The method according to any one of claims 1 to 5, wherein the MR data comprise, for each object of the plurality of objects, first MR data, and second MR data,
- wherein the first MR data represent a first examination region within the object after administration of the first dose of the contrast agent, wherein the first MR data were acquired according to a first MR sequence protocol,
- wherein, the second MR data represent a second examination region within the object after the administration of the first dose of the contrast agent, wherein the second MR data were acquired according to the first MR sequence protocol or a second MR sequence protocol,
- wherein the target MR data represent the first examination region within the object after administration of a second dose of a contrast agent, wherein the second dose is different from the first dose,
- wherein the training of the machine learning model comprises:
∘ inputting the first MR data, and the second MR data into the machine learning model,
wherein the machine learning model is configured to generate, at least partially on the basis of the first MR data, the second MR data, and model parameters, predicted MR data.

7. The method according to any one of claims 1 to 5, wherein the MR data comprise, for each object of the plurality of objects, first MR data, and second MR data,
- wherein the first MR data represent a first examination region within the object after administration of the first dose of the contrast agent, wherein the first MR data were acquired according to a first MR sequence protocol,
- wherein the second MR data represent a number of additional examination regions within the object after the administration of the first dose of the contrast agent, wherein the additional examination regions together with the first examination region form a stack of adjacent and/or partially overlapping slices, wherein the second MR data were acquired according to the first MR sequence protocol or a second MR sequence protocol,
- wherein the training of the machine learning model comprises:
∘ inputting the first MR data, and the second MR data into the machine learning model,
wherein the machine learning model is configured to generate, at least partially on the basis of the first MR data, the second MR data, and model parameters, predicted MR data.

8. The method according to any one of claims 1 to 5, wherein the MR data comprise, for each object of the plurality of objects, first MR data, second MR data, and third MR data,
- wherein the first MR data represent a first examination region within the object after administration of the first dose of the contrast agent, wherein the first MR data were acquired according to a first MR sequence protocol, wherein the first MR data preferably comprise more than one representation of the first examination region,
- wherein the second MR data represent a number of additional examination regions within the object after the administration of the first dose of the contrast agent, wherein the additional examination regions together with the first examination region form a stack of adjacent and/or partially overlapping slices, wherein the second MR data were acquired according to the first MR sequence protocol or a second MR sequence protocol, wherein the second MR data preferably comprise more than one representation of at least some of the additional examination regions,
- wherein the third MR data, if present, represent the first examination region and/or the second examination region without a contrast agent, wherein the third MR data were acquired according to the first MR sequence protocol or the second MR sequence protocol or a third sequence protocol,
- wherein the training of the machine learning model comprises:
∘ inputting the first MR data, the second MR data, and optionally the third MR data into the machine learning model,
wherein the machine learning model is configured to generate, at least partially on the basis of the first MR data, the second MR data, optionally the third MR data and model parameters, predicted MR data.

9. The method according to any one of claims 4 to 8,
- wherein the first MR data comprise an in-phase MR image, an opposed phase MR image, a water-only MR image, and a fat-only MR image, and/or
- wherein the second MR data comprise an opposed phase MR image, a water-only MR image, and a fat-only MR image, and/or
- wherein the third MR data, if present, comprise a water-only MR image, and/or
- wherein the first MR sequence protocol is a T1-weigthed DIXON-type sequence protocol, more preferably a T1-VIBE-DIXON-type sequence protocol, and/or
- wherein the second sequence protocol is a T1-weigthed DIXON-type sequence protocol, more preferably a T1-VIBE-DIXON-type sequence protocol.

10. The method according to any one of claims 1 to 9,
- wherein the second dose is higher than the first dose,
- wherein the first dose preferably is a dose which is equal to or less than the dose which is recommended by the manufacturer or distributor of the contrast agent and/or the first dose is preferably equal to or less than a standard dose approved by an authority for an MR examination,
- wherein the second dose is preferably the dose which is recommended by the manufacturer or distributor of the contrast agent and/or the dose which is mentioned in the product label of the contrast agent and/or the dose approved by an authority for an MR examination (also referred to as standard dose), or the second dose is preferably a dose that is required to obtain a defined appearance of the (first) examination region.

11. A computer system comprising:
a processor; and
a memory storing an application program configured to perform, when executed by the processor, an operation, the operation comprising:
- receiving a training data set, the training data set comprising, for each object of a multitude of objects, MR data, and target MR data,
- wherein the MR data comprise a plurality of representations of a first examination region within the object after administration of a first dose of a contrast agent,
- wherein the target MR data represent at least a portion of the first examination region within the object after administration of a second dose of a contrast agent, wherein the second dose is different from the first dose,
- training the machine learning model, thereby obtaining a trained machine learning mode, wherein the training comprises:
∘ inputting the MR data into the machine learning model,
wherein the machine learning model is configured to generate, at least partially on the basis of the MR data and model parameters, predicted MR data,
∘ receiving from the machine learning model the predicted MR data,
∘ computing a loss value, the loss value quantifying the deviations between the predicted MR data and the target MR data,
∘ modifying one or more of the model parameters to minimize the loss value,
- outputting the trained machine learning model, and/or storing the machine learning model on a data storage, and/or providing the trained machine learning model for predictive purposes.

12. A non-transitory computer readable medium having stored thereon software instructions that, when executed by a processor of a computer system, cause the computer system to execute the following steps:
- receiving a training data set, the training data set comprising, for each object of a multitude of objects, MR data, and target MR data,
- wherein the MR data comprise a plurality of representations of a first examination region within the object after administration of a first dose of a contrast agent,
- wherein the target MR data represent at least a portion of the first examination region within the object after administration of a second dose of a contrast agent, wherein the second dose is different from the first dose,
- training the machine learning model, thereby obtaining a trained machine learning mode, wherein the training comprises:
∘ inputting the MR data into the machine learning model,
wherein the machine learning model is configured to generate, at least partially on the basis of the MR data and model parameters, predicted MR data,
∘ receiving from the machine learning model the predicted MR data,
∘ computing a loss value, the loss value quantifying the deviations between the predicted MR data and the target MR data,
∘ modifying one or more of the model parameters to minimize the loss value,
- outputting the trained machine learning model, and/or storing the machine learning model on a data storage, and/or providing the trained machine learning model for predictive purposes.

13. Kit comprising a contrast agent and a non-transitory computer-readable storage medium according to claim 12.

14. Kit according to claim 13, wherein the magnetic resonance contrast agent is selected from the group of gadolinium chelates, gadobenate dimeglumine, gadoteric acid, gadodiamide, gadoteridol, and gadobutrol.

15. Kit according to any one of the claims 13 to 14, wherein the magnetic resonance contrast agent is a substance or a substance mixture with gadoxetic acid or a gadoxetic acid salt as contrast-enhancing active substance, preferably the disodium salt of gadoxetic acid.
